(19) Europäisches Patentamt
European Patent Office
Office européen des brevets

(11) Numéro de publication: **0 270 460 B1**

(12) # FASCICULE DE BREVET EUROPEEN

(45) Date de publication de fascicule du brevet: **19.04.95**

(51) Int. Cl.6: **A61K 47/48**, A61K 9/127

(21) Numéro de dépôt: **87402747.7**

(22) Date de dépôt: **03.12.87**

Le dossier contient des informations techniques présentées postérieurement au dépôt de la demande et ne figurant pas dans le présent fascicule.

(54) **Microcristaux comportant une substance active présentant une affinité pour les phospholipides et au moins un phospholipide, procédé de préparation.**

(30) Priorité: **05.12.86 FR 8617116**
**19.02.87 FR 8702137**
**08.09.87 FR 8712424**

(43) Date de publication de la demande:
**08.06.88 Bulletin 88/23**

(45) Mention de la délivrance du brevet:
**19.04.95 Bulletin 95/16**

(84) Etats contractants désignés:
**AT BE CH DE ES FR GB GR IT LI LU NL SE**

(56) Documents cités:
**EP-A- 0 224 457**
**EP-A- 0 260 811**
**WO-A-87/01933**
**WO-A-89/03208**
**US-A- 4 663 167**

(73) Titulaire: **THE LIPOSOME COMPANY, INC.**
**One Research Way**
**Princeton Forrestal Center**
**Princeton, NJ 08540 (US)**

(72) Inventeur: **Baurain, Roger**
**Av. des Violettes 72**
**B-1970 Wezembeek**
**Oppem (BE)**
Inventeur: **Trouet, André**
**29 Predikherenberg**
**B-3009 Winksele**
**Herent (BE)**

(74) Mandataire: **Warcoin, Jacques et al**
**Cabinet Régimbeau,**
**26, avenue Kléber**
**F-75116 Paris (FR)**

EP 0 270 460 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

JOURNAL OF PHARMACEUTICAL SCIENCES, vol. 73, no. 6, juin 1984, pages 757-761,American Pharmaceutical Association; S. VENKATARAM et al.: "Characteristics ofdrug-phospholipid coprecipitates I: Physical properties and dissolutionbehavior of griseofulvin-dimyristoylphosphatidylcholine systems"

SERVEUR S.T.N. (KARLSRUHE),(BASE CA): CHEMICAL ABSTRACTS, vol. 95, no. 16,abstract no. 138545z, Columbus, Ohio, US;K.-Y. WENG et al.: "Drug carrier - studyon liposome of gossypol",& YAO HSUEH T'UNG PAO, 16(3), 59

SERVEUR S.T.N. (KARLSRUHE),(BASE CA): CHEMICAL ABSTRACTS, vol. 107, no. 5,abstract no. 33378k, Columbus, Ohio, US;C. TOUVAY et al.: "Gossypol: a potentinhibitor of PAF-acether- andleukotriene-induced contractions ofguinea pig lung parenchyma strips", J.PHARMACOL., 39(6), 454-8

Liposomes, edited by Marc J. Ostro (1983), Marcel Dekker Inc N.Y. and Basel, p.53, 66 and 67

## Description

La présente invention concerne des microparticules lipidiques d'aspect cristallin appelées microcristaux. Il s'agit de microcristaux d'une substance insoluble dans l'eau présentant une affinité pour les phospholipides,et d'au moins un phospholipide.

La présente invention concerne également un procédé de préparation de microcristaux d'une substance insoluble dans l'eau présentant une affinité pour les phospholipides. Elle concerne les microcristaux obtenus, ainsi que les compositions pharmaceutiques en comprenant.

Le procédé est caractérisé en ce qu'il comporte les étapes suivantes :

a) on évapore le ou les solvant(s) d'une solution de phospholipide(s) et de ladite substance,

b) on remet le film obtenu après évaporation du ou des solvant(s), en suspension dans une solution aqueuse après agitation vigoureuse.

Dans la présente demande, on entend par "microcristaux" les microparticules lipidiques d'aspect cristallin obtenues notamment par le procédé ci-dessus ou un procédé qui en dérive, étant entendu que la structure cristalline n'est pas nécessairement obtenue stricto sensu.

Dans ce procédé, il faut entendre par "substance insoluble dans l'eau", une substance peu ou pas soluble dans l'eau et par "substance présentant une affinité pour les phospholipides" un composé chimique plus particulièrement capable d'interagir avec les phospholipides par voie physique. On en donnera des exemples par la suite.

L'article du Journal of Pharmaceutical Sciences, vol. 73 (1984), N̲o̲ 6, décrit des microcristaux de griséofulvine, et de dimyristolylphosphatidylcholine - de faible stabilite. En outre, aucune application thérapeutique avantageuse n'y est décrite.

Les microcristaux, obtenus selon l'invention, offrent principalement l'avantage d'obtenir une microsuspension stable en solution aqueuse de microcristaux d'une substance par ailleurs insoluble, ce qui permet d'administrer ladite substance, lorsqu'il s'agit d'un médicament, sous forme injectable ou pulvérisable. Comparées aux liposomes, les préparations sous forme de microcristaux, selon l'invention, sont tout d'abord plus simples et beaucoup plus économiques à préparer dans la mesure où ils impliquent la présence de constituants en nombre réduit. Ils sont beaucoup plus stables, alors même que l'instabilité des liposomes en limite considérablement les conditions d'emploi.

En outre, vraisemblablement en vertu d'un effet de pilotage accru notamment vers les macrophages d'une part, et d'un effet de libération progressive de la substance active incorporée, d'autre part, les préparations de microcristaux donnent des résultats d'activité thérapeutique et de toxicité également nettement plus intéressants que les vésicules du type liposome.

En général, selon l'invention, on utilisera de préférence comme phospholipide, de la phosphatidylcholine. Celle-ci toutefois peut être utilisée éventuellement en combinaison avec d'autres éléments connus, par exemple dans la préparation des liposomes, à savoir des stérols comme le cholestérol ou encore de la stéarylamine.

Cependant, s'agissant de la formation de microcristaux, le rapport molaire (phospholipide(s)/substance) est d'une influence déterminante vis-à-vis notamment du rapport molaire (phospholipide(s)/stérol(s)) par exemple,qui n'a pas une influence sur celle-ci.

Dans certains cas,cependant, il peut être intéressant d'ajouter un ou des stérol(s) pour renforcer l'activité de la substance active insoluble à incorporer.

Le rapport molaire (phospholipide(s)/substance) doit être inférieur à celui au-delà duquel on observe uniquement des vésicules de type liposome.

En effet, si le rapport molaire (phospholipide(s)/substance) engagé dans le procédé selon l'invention est trop élevé, notamment supérieur à 10 par exemple, on observe essentiellement la formation de vésicules lipidiques ressemblant à des liposomes.

En revanche, si le rapport molaire est inférieur ou égal à 2, de préférence voisin de 1, on observe une interaction spécifique avec production homogène de microcristaux dont la taille moyenne est comprise entre 0,1 $\mu$m et 1 $\mu$m.

Pour des rapports intermédiaires,des mélanges de vésicules lipidiques et de microcristaux sont obtenus.

Le rapport molaire entre le ou les phospholipide(s) ou un ou des stérol(s), le cas échéant, comme le cholestérol, n'a pas une influence déterminante sur la formation de microcristaux. Toutefois, dans certains cas il peut être intéressant d'ajouter un ou des stérol(s) pour renforcer l'activité de la substance active.

Selon une variante du procédé, l'étape a) du procédé se subdivise ainsi :

a1) on évapore le ou les solvant(s) de la solution de phospholipide(s),

a2) on remet le film lipidique obtenu après évaporation, en solution, en ajoutant une solution de ladite substance,

a3) on évapore le ou les solvant(s) de la solution de phospholipide(s) et de ladite substance.

Dans le procédé, le rapport molaire entre le ou les phospholipide(s) d'une part, et ladite substance d'autre part, est de préférence compris entre 0,1 et 2 et de préférence compris entre 0,8 et 1,2 ou encore voisin de 1.

Lorsqu'on associe un stérol à la substance à traiter, le rapport molaire dans le procédé entre le ou les phospholipide(s) et le ou les stérol(s) est de préférence compris entre 1 et 2.

Pour remettre le film obtenu à l'étape b) en suspension, ceci peut être effectué par diverses techniques connues, mais il est intéressant de pouvoir utiliser le traitement aux ultrasons.

Dans un mode de réalisation particulier de l'invention, les solvants utilisés sont des solvants organiques usuels, tels que le chloroforme ou le méthanol ou encore des mélanges de solvants, par exemple de chloroforme et de méthanol.

Par exemple, la solution de phospholipide est une solution de chloroforme et la solution de la substance en cause est une solution de mélange chloroforme/méthanol. La solution de stérol, le cas échéant, peut être une solution de méthanol.

Selon une autre caractéristique du procédé, les microcristaux sont purifiés par centrifugation et lavage à l'eau distillée ou avec une solution aqueuse.

La présente invention concerne, par exemple, des microcristaux comportant à titre de substance active des ginkgolides.

Les plus connus des ginkgolides sont les ginkgolides A, B, C et M qui sont utiles en thérapeutique pour le traitement et la prévention des maladies provoquées par le PAF-acéther comme cela est décrit dans le brevet Belge 901915. Parmi ces composés le ginkgolide B s'est révélé être particulièrement intéressant.

Le ginkgolide B est très peu soluble ; ceci constitue un inconvénient lorsque l'on veut l'incorporer dans certaines compositions pharmaceutiques en particulier des compositions injectables. De plus, il est intéressant d'avoir à sa disposition des compositions pharmaceutiques capables de convoyer la substance active jusqu'aux sites d'activité et d'accroître ainsi la spécificité de la substance active tout en réduisant dans la mesure du possible les effets indésirables.

La présente invention vise à résoudre les problèmes énoncés ci-dessus avec la nouvelle forme cristalline appelée "microcristaux".

Dans le cas particulier évoqué, les substances insolubles selon l'invention seront choisies parmi les ginkgolides et leurs dérivés et plus particulièrement il s'agira du ginkgolide B. Bien que l'on préfère parmi les ginkgolides le ginkgolide B, il est également possible d'utiliser les dérivés, tels que les monoacétates, les triacétates, les dérivés tétrahydro- ou acétylés.

Les ginkgolides sont des substances qui interviennent dans le traitement des maladies provoquées par l'acéther du "Facteur d'activation des plaquettes".

L'acéther du "Facteur d'activation des plaquettes" (PAF-acéther) est un phospholipide qui peut être la cause de nombreuses maladies chez l'être humain ou chez les animaux. L'acéther du "Facteur d'activation des plaquettes" provoque l'agrégation des plaquettes, ainsi que la libération de leur amine baso-active. La libération est provoquée chez les animaux et les êtres humains sous l'effet de différents types de chocs, tels que les chocs anaphylactiques, les brûlures, les chocs septiques, les chocs par irradiation et les traumas. Sa libération conduit par la suite à une suppression des réactions immunitaires suite à l'épuisement des moyens de défense de l'organisme. En effet, depuis son identification (1-0-alkyl-2(R)-acétyl-glycéro-3-phosphorylcholine) et sa synthèse totale, les preuves de sa participation dans certains processus physiopathologiques, se sont accumulées, principalement dans l'anaphylaxie pulmonaire et différents états de choc.

On connaît de nombreux antagonistes spécifiques du PAF-acéther. On en distingue principalement deux séries :

1) des produits présentant une structure analogue à celle du PAF-acéther,

2) des produits naturels à activité antagoniste spécifique.

Parmi cette deuxième catégorie, l'on trouve d'une part les lignanes et néolignanes, tels que la kadsurénone (Piper futokadsurae), la magnosalicine (Magnolia salicifolia), les nectandrines A et B (Nectandra rigida) et le dinorlignane de synthèse L-652.731 et, d'autre part, les terpénoïdes isolés du Ginkgo biloba (Ginkgolides).

Cependant, ces substances antagonistes de l'acéther du "Facteur d'activation des plaquettes" présentent un inconvénient majeur, en ce qu'ils sont très peu solubles, ce qui rend leur utilisation en fait quasiment inexistante car subordonnée dans la pratique à une administration par perfusion.

De manière tout à fait surprenante, on a découvert, selon l'invention, que les ginkgolides peuvent être rendus injectables en interagissant avec des phospholipides sous forme de microcristaux.

Il en est de même pour les autres substances antagonistes de l'acéther du "Facteur d'activation des plaquettes", substances hydrophobe et interagissant avec les phospholipides. La plupart de ces substances se sont en effet avérées d'une part, hydrophobe et d'autre part, présentant également une affinité pour les phospholipides et donc une aptitude à la formation de microcristaux avec les phospholipides, ces microcristaux constituant une microsuspension stable en solution aqueuse.

L'affinité des substances antagonistes de l'acéther du "Facteur d'activation des plaquettes" pour les phospholipides est sans doute à l'origine de leur action inhibitrice sur le PAF-acéther qui présente, comme on l'a vu, également une structure phospholipidique. Comme il a été découvert par la demanderesse, cette affinité offre la possibilité d'injecter ces substances par la préparation de microcristaux. Mais, en outre, il est possible que la similitude des structures phospholipidiques d'une part, des microcristaux formés et, d'autre part, du PAF-acéther contribue également à une plus grande activité thérapeutique des compositions pharmaceutiques contenant ces microcristaux de substances antagonistes du PAF-acéther.

Selon un autre exemple de la présente invention, la substance active en cause peut donc être choisie parmi les substances antagonistes de l'acéther du "Facteur d'activation des plaquettes".

Dans ce procédé, il faut entendre par substances antagonistes de l'acéther du "Facteur d'activation des plaquettes" une substance permettant de traiter efficacement les maladies provoquées par l'acéther du "Facteur d'activation des plaquettes".

Plus particulièrement, selon la présente invention, les substances antagonistes de l'acéther du "Facteur d'activation des plaquettes" peuvent être choisies parmi des produits de synthèse présentant une structure analogue à celle du "Facteur d'activation des plaquettes" ou des produits naturels, tels que les terpénoïdes, les lignanes et les néolignanes.

Notamment parmi les lignanes et néolignanes, les substances antagonistes de l'acéther du "Facteur d'activation des plaquettes" sont choisies parmi la kadsurénone, la magnosalicine, les nectandrines A et B et le dinorlignane de synthèse L-652.731.

Parmi les produits de synthèse, on peut citer les dérivés de canthène 5, 6.

Selon un autre exemple de la présente invention, la substance active en cause peut être choisie parmi l'amphotéricine B et ses dérivés présentant une activité antifongique. On trouvera une description de certains de ces composés dans la littérature [1].

L'amphotéricine B et ses dérivés, notamment dérivés aminoacyl, continuent à être les médicaments de choix pour le traitement de nombreuses infections fongiques profondes en dépit de leur toxicité sévère et d'effets secondaires néfastes importants.

Il a déjà été proposé pour réduire la toxicité et augmenter l'activité de l'amphotéricine B de l'encapsuler dans des liposomes. Cependant les résultats obtenus, à ce point de vue, quoique positifs, ne sont pas encore suffisants.

Les microcristaux d'amphotéricine B ou de ses dérivés permettent de pallier à ces inconvénients de toxicité importante, tout en augmentant l'activité antifongique desdits substances, de manière nettement plus efficace que les liposomes, vraisemblablement entre autres en vertu d'un effet de pilotage de la substance vers les cellules cibles comme les macrophages, ainsi qu'un effet de libération progressive de la substance active améliorés. En outre, le procédé de préparation de microcristaux est, d'une manière générale, plus simple à réaliser que l'encapsulation dans des liposomes et les préparations obtenues sont plus stables.

La préparation de microcristaux permet également d'injecter l'amphotéricine $\beta$ sans usage du déoxycholate comme dans la fungizone ®, celui-ci étant toxique.

L'amphotéricine présente une affinité pour les stérols, c'est pourquoi, dans un mode de réalisation du procédé selon la présente invention, l'amphotéricine B ou un de ses dérivés sont additionnés de stérol(s), notamment du cholestérol (Ch) ou de l'ergostérol.

De manière avantageuse dans l'étape B du procédé, lorsque la substance en cause est un agent anti-PAF-acéther, on remet le film obtenu après évaporation du solvant en suspension dans une solution tamponnée à pH compris entre 5 et 8, de préférence à pH 5,9, par exemple dans un tampon acétate ou phosphate.

Dans l'étape b) du procédé, lorsque la substance est l'amphotéricine B, notamment en l'absence de stérol, la solution aqueuse est par exemple une solution de NaCl. En présence de stérol, la solution aqueuse de l'étape b) du procédé peut être de l'eau distillée ou du NaCl. Alors avantageusement après l'étape b) du procédé, les microcristaux sont purifiés par centrifugation et lavage en solution aqueuse de l'étape B.

On peut également avantageusement notamment lorsque la substance en cause est de l'amphotéricine B utiliser comme solution aqueuse des solutions de saccharides, par exemple le lactose ou encore le glucose, notamment des solutions 0,1 M. Les microcristaux obtenus peuvent, dans ces conditions, être lyophilisés. Il en résulte une possibilité de conservation à long terme.

La présente invention a également pour objet les microcristaux obtenus et notamment des préparations de microcristaux qui peuvent être lyophilisés.

Il s'agit de microcristaux d'une substance insoluble dans l'eau et présentant une affinité pour les phospholipides et au moins un phospholipide, dans un rapport molaire (phospholipide(s)/substance insoluble) est inférieur ou égal à 2 et notamment est compris entre 0,8 et 1,4 ou encore voisin de 1.

Ces microcristaux ont une taille comprise entre 0,1 $\mu$m et 2 $\mu$m, ou plus particulièrement voisin de 500 nm.

Les microcristaux selon l'invention peuvent comporter par exemple une substance insoluble choisie parmi les ginkgolides et leurs dérivés et un phospholipide qui peut être la phosphatidylcholine ou un de ses dérivés.

Par exemple on citera des microcristaux, caractérisés en ce qu'ils comportent du ginkgolide B et de la phosphatidylcholine dans un rapport sensiblement équimolaire.

Plus généralement, l'invention a également pour objet des microcristaux contenant un agent anti-PAF-acéther et un ou des phospholipide(s) de préférence dans un rapport molaire voisin de 1.

Selon un autre exemple, la présente invention a pour objet des microcristaux contenant de l'amphotéricine B ou d'un de ses dérivés antifongiques.

Dans un mode de réalisation particulier, ces microcristaux comportent de l'amphotéricine B ou un de ses dérivés, additionnée ou non de stérol(s) et de la phosphatidylcholine.

Par exemple, les microcristaux comportent de l'amphotéricine B et de la phosphatidylcholine dans un rapport molaire voisin de 1.

Lorsque l'on associe un stérol à la substance active, du point de vue du rapport molaire des composants incorporés dans le microcristaux, le rapport molaire (phospholipide(s)/stérol(s) + substance) compris entre 0,8 et 1,4 ou encore voisin de 1.

Ainsi, on peut citer notamment le rapport molaire (phosphatidylcholine/cholestérol/amphotéricine B) de 4/2/1 (soit un rapport molaire global entre le phospholipide et la substance à traiter additionnés de stérol(s)) de 4/3.

La présente invention a également pour objet des compositions pharmaceutiques comportant à titre de principes actifs des microcristaux selon la présente invention.

De préférence, ces compositions pharmaceutiques sont conditionnées sous forme injectable ou pulvérisable ou encore sous forme lyophylisée.

D'autres caractéristiques et avantages de la présente invention apparaîtront à la lumière de la description détaillée qui va suivre sous forme d'exemples.

## EXEMPLE 1

## MISE AU POINT DE FORMES ADMINISTRABLES DU GINGKOLIDE B

Les gingkolides A, B et C sont des terpènes isolés des feuilles de Gingko biloba et possédant une activité antagoniste vis-à-vis de l'acéther du facteur d'activation des plaquettes, phospholipide impliqué dans les réactions inflammatoires et d'hypersensibilité immédiate (par exemple agrégation plaquettaire, spasmes bronchiques, ...).

Le composé le plus actif : le gingkolide B a une solubilité limitée dans l'eau à 100 $\mu$g/ml. Par contre, le traitement ou la prévention des maladies provoquées par l'acéther du facteur d'activation des plaquettes implique l'administration de 1 à 50 mg de gingkolide par kg soit pour 1 homme de 70 kg, entre 70 et 3500 mg de gingkolide B.

Il se posait donc le problème de l'obtention d'une forme administrable du gingkolide B. La molécule étant très hydrophobe, on a tout d'abord pensé à enrober le gingkolide B dans des vésicules lipidiques (liposomes) et ultérieurement, une méthode d'obtention de microcristaux de gingkolide B associés à de la phosphatidylcholine a été développée donnant de meilleurs résultats.

### A) INCORPORATION DANS DES LIPOSOMES

Il a d'abord été choisi une incorporation dans des liposomes multilamellaires composés de phosphatidylcholine (PC), cholestérol (Ch) et de stéarylamine (SA)

Les liposomes ont été préparés à pH 7,4 puis à pH légèrement acide.

1. Préparation de liposomes

Des liposomes multilamellaires contenant le gingkolide B ont été préparés comme suit.

Les lipides : 50 µmoles (39,25 mg) de phosphatidylcholine (PC), 37,5 µmoles (14,51 mg) de cholestérol (Ch) et 12,5 µmoles (3,37 mg) de stéarylamine (SA) sont dissous dans 10 ml de chloroforme-méthanol (4:1 en volume) dans un ballon de 500 ml. La gingkolide B dissoute dans le méthanol y est ajoutée (0,86 à 4,25 mg soit 2-10 µmoles) et le solvant est évaporé sous vide à l'aide d'un évaporateur rotatif à une température de 30 °C. Le film est repris dans 10 ml de chloroforme et réévaporé pour obtenir un film lipidique uniforme dans le ballon.

Les liposomes multilamellaires (MLV) sont formés par addition de 4 ml de tampon phosphate pH 7,4, 0,15 M en NaCl et agitation vigoureuse au vortex pendant 5 minutes puis laissés sous azote pendant 16 heures.

L'efficacité de l'incorporation du gingkolide B dans les liposomes MLV dépend de la quantité de gingkolide B engagée et atteint à pH 7,4 un maximum de 64 %.

Une étude de la stabilité de ces liposomes contenant le gingkolide B indique qu'à chaque lavage des MLV, on perd jusqu'à environ 100 µg de gingkolide B.

2. Liposomes préparés à pH 6,5

Le gingkolide B a été incorporé dans des liposomes MLV composés de phosphatidylcholine, de cholestérol et de stearylamine (4:3:1) à pH 6,5 (tampon phosphate 0,15 M) suivant la même procédure qu'à pH 7,4, mais en augmentant la quantité de gingkolide mise en présence des lipides. Les résultats indiquent que le pourcentage d'incorporation atteint 87 %, soit 1 molécule de gingkolide B pour 4,4 molécules de phosphatidylcholine.

3. Liposomes préparés à pH 5,9

Le gingkolide B a été incorporé dans les liposomes MLV à pH 5,9 suivant la même procédure qu'à pH 7,4. Les résultats montrent que le pourcentage d'incorporation est amélioré encore soit 1 molécule de G-B pour 2,8 molécules de PC. La meilleure incorporation à pH 5,9 s'explique sans doute par la plus grande stabilité du gingkolide B à ce pH, l'ouverture des cycles lactones à pH neutre ou alcalin, conduisant à des produits plus hydrophiles étant connue. Toutefois, la stabilité des liposomes n'est pas améliorée.

B) PREPARATION DE MICROCRISTAUX DE GINGKOLIDE B

En plus de liposomes, on a observé la présence de microcristaux stables dans une préparation dont le rapport molaire PC/GB était 1,54.

L'observation de microcristaux à la place de liposomes contenant le gingkolide B a conduit à étudier les conditions de leur obtention maximum. On a tout d'abord analysé l'influence, sur leur formation, de la composition lipidique des liposomes et celle du rapport entre la quantité de phosphatidylcholine et de gingkolide B.

1. Microcristaux obtenus à partir de PC, Ch et de SA

Dans un premier temps, on a fait varier les proportions de phosphatidylcholine (PC), de cholestérol (Ch) et de stéarylamine (SA). Cependant, les rapports molaires PC/GB ne sont pas semblables et il ressort des résultats, présentés au tableau I ci-après, que le rapport entre la phosphatidylcholine et le cholestérol n'a pas une influence déterminante sur la formation de microcristaux. Par contre, le rapport PC/GB est essentiel et a donc été étudié dans un deuxième temps.

Des préparations du type "liposome" composés de PC:Ch:SA dans un rapport (5:4:1) ont été obtenues avec un rapport PC/GB de 7,5 et plus (préparation E-31). L'examen en microscopie optique à contraste de phase montre que la préparation après sonication est constituée essentiellement de liposomes avec de rares microcristaux de même taille que les liposomes.

L'influence du rapport PC/GB sur la formation de microcristaux contenant le gingkolide B est détaillée au tableau II ci-après pour des mélanges lipidiques composés de PC:Ch:SA dans un rapport molaire 4:3:1. Il en ressort que si le rapport PC/GB est supérieur à 7,5, on observe essentiellement la formation de

vésicules lipidiques ressemblant aux liposomes tandis que si le rapport molaire est inférieur ou égal à 2, de préférence voisin de 1, on observe principalement des microcristaux dont la taille moyenne est comprise entre 0,2 et 0,7 µm. Pour des rapports PC/GB intermédiaires, des mélanges de liposomes et de microcristaux sont obtenus.

Ces microcristaux sont beaucoup plus petits que ceux obtenus en traitant du gingkolide B seul dans les mêmes conditions.

2. Toxicité

Des aliquots des préparations E-31 (liposomes) et E-34 (microcristaux) ont été conservés pour une étude de toxicité aiguë chez la souris.

La préparation E-31 (liposomes) injectée en i.v. à des souris à la dose de 13,1 et 25,9 mg/kg induit une perte de poids de 1,8 et 7,1 % respectivement après 12 jours. Par contre, la préparation E-34 (microcristaux) n'est toxique qu'à la dose de 48,9 mg/kg (souris morte 2 heures après l'injection i.v.) tandis qu'à 21.8 mg/kg, aucune toxicité n'est observée, au contraire, on observe un gain de poids de 24 % après 12 jours.

EXEMPLE 2

PREPARATION DE MICROCRISTAUX DE GINGKOLIDE B PAR INTERACTION PREFERENTIELLE AVEC LA PHOSPHATIDYLCHOLINE

L'obtention de microcristaux par interaction entre le gingkolide B et les constituants habituellement utilisés pour la préparation de liposomes a conduit à ôter chaque fois un de ces constituants et de déterminer la façon la plus simple de les préparer. Il apparaît que la phosphatidylcholine est essentielle, mais que la présence du cholestérol et de la stéarylamine ne l'est pas pour l'obtention des microcristaux, d'où une grande simplification du procédé.

Si on prépare des microcristaux ayant un rapport molaire PC/GB de 1,0 sans cholestérol, on obtient de petits microcristaux de tailles et de formes homogènes. Les microcristaux préparés de la même façon à partir de PC et de Ch, sans SA et ayant un rapport molaire PC/GB de 1,0 ont tendance à s'agréger et leur taille moyenne est supérieure à 4 µm.

1. Microcristaux PC:GB

Finalement, si on enlève le cholestérol et la stéarylamine, il ne reste que la phosphatidylcholine et le gingkolide B. Si le rapport molaire PC/GB est inférieur ou égal à 2,0, on obtient uniquement des microcristaux.

Tel qu'indiqué au tableau III ci-après, si le rapport molaire PC/GB est supérieur à 2,0, on observe la présence de microvésicules lipidiques ("liposomes") dans la préparation, dont la taille moyenne est supérieure à 1 µm. Si le rapport molaire PC/GB est inférieur à 1,0, nous observons une population de microcristaux hétérogènes au point de vue taille et forme, mais dont la taille moyenne est supérieure à 1 µm.

Pour un rapport PC/GB equimolaire, nous obtenons des microcristaux plus petits, de taille moyenne comprise entre 0,2 et 0,7 µm. Leur formation résulte d'une interaction spécifique entre le gingkolide B et la phosphatidylcholine.

Préparation de microcristaux à pH 5,9 (E-35)

Les microcristaux contenant le gingkolide B (GB) sont préparés à partir de 0,10 mmoles de PC et 0,10 mmoles de gingkolide B.

a) Préparation

Le film lipidique est formé dans un ballon de 500 ml en dissolvant 78,5 mg de PC dans 10 ml de chloroforme-méthanol (4:1 en volume) et 42,5 mg de gingkolide B dans 50 ml de méthanol et en évaporant le solvant sous vide à l'aide d'un évaporateur rotatif à une température de 30 °C.

Les microcristaux sont formés par addition de 50 ml de tampon acétate 0,15 M pH 5,9 et sonication pendant 5 min. à 100 Watts dans un bain à ultra-sons. Les microcristaux GB:PC sont conservés sous azote à 4 °C.

b) Purification

La préparation de microcristaux est centrifugée à 2000 g pendant 15 min. et les microcristaux sont

lavés 2 fois avec de l'eau bidistillée. Les microcristaux dans l'eau ont le même aspect microscopique que ceux obtenus dans du tampon acétate à pH 5,9.

On obtient une suspension de microcristaux de Ginkgolide B ayant une taille homogène de l'ordre de 500 nm à une concentration en gingkolide B de 12,8 mg/ml.

2) Activité anti-PAF-a-acéther

On a comparé le gingkolide B à 2 préparations de liposomes contenant du gingkolide B (préparation E-30 et E-31) ainsi qu'à une préparation de microcristaux (préparation E-34) dans des tests ex vivo sur le lapin en pourcentage d'inhibition sur l'agrégation plaquettaire (PAF 2,5 nM).

Il en ressort que les préparations à base de liposomes n'ont un effet inhibiteur sur l'agrégation des plaquettes essentiellement jusqu'à 1h 30 seulement, tandis que l'effet des microcristaux était plus prolongé à des doses de 1 mg/kg administrées en i.v.

E-30 = Liposomes (PC/CH/SA) = (7/2/1) à 1,283 mg GB/ml
E-31 = Liposomes (PC/CH/SA) = (5/4/1) 2,695 mg GB/ml
E-34 = microcristaux avec 4,250 mg GB/ml
tampon = tampon acétate PH 6.0 ; 0,15 M.

TABLEAU I : EFFET DE LA COMPOSITION LIPIDIQUE SUR L'OBTENTION DE MICROCRISTAUX CONTENANT DU GINGKOLIDE B

| Composition | Rapport Molaire (PC/GB) | Taille moyenne (nm) | Aspect en microscopie optique à contraste de phase |
|---|---|---|---|
| PC:Ch:SA (7:2:1) | 3.1* | N.D. | Liposomes ronds et allongés, pas de microcristaux |
| PC:Ch:SA (4:3:1) | 1.25 | 634 nm | Microcristaux, peu ou pas de liposomes |
| PC:Ch:SA (5:4:1) | 7.5** | N.D. | Liposomes (> 95 %) + quelques microcristaux |
| PC:Ch:SA (4:6:1) | 1.0 | > 4000 nm | Microcristaux allongés + gros cristaux de cholestérol |

* préparation E-30
** préparation E-31
N.D. = non déterminé

TABLEAU II : EFFET DU RAPPORT MOLAIRE PC/GB SUR L'OBTENTION DE MICROCRISTAUX CONTENANT DU GINGKOLIDE B

| Composition | Rapport Molaire (PC/GB) | Taille Moyenne (nm) | Aspect en microscopie optique à contraste de phase |
|---|---|---|---|
| PC:Ch:SA (4:3:1) | 10.0 | N.D. | Essentiellement des petits liposomes |
| PC:Ch:SA (4:3:1) | 7.5 | N.D. | Liposomes ronds et allongés + rares microcristaux (1-5 %) |
| PC:Ch:SA (4:3:1) | 5.0 | N.D. | Liposomes + microcristaux (10-25 %) |
| PC:Ch:SA (4:3:1) | 2.5 | N.D. | Microcristaux (50-70 %) + liposomes |
| PC:Ch:SA (4:3:1) | 1.25 | 634 nm | Principalement de petits microcristaux + rares liposomes (1-5 %) |
| PC:Ch:SA (4:3:1) | 0.60* | 242 nm | Essentiellement des microcristaux de taille ± homogène |

* préparation E-34

TABLEAU III: EFFET DU RAPPORT MOLAIRE PC/GB SUR L'OBTENTION DE MICROCRISTAUX CONTENANT DU GINGKOLIDE B

| Composition | Rapport Molaire (PC/GB) | Taille Moyenne (nm) | Aspect en microscopie optique à contraste de phase |
|---|---|---|---|
| PC:GB | 4.0* | 2100 | - Liposomes + agrégats de liposomes<br>- peu de microcristaux |
| PC:GB | 2.0 | 2200 | Mélange de liposomes petits et agrégés et de microcristaux |
| PC:GB | 1.0** | 250 à 650 | Petits microcristaux, pas de liposomes |
| PC:GB | 0.5 | 2970 | Petits microcristaux et population hétérogène de cristaux plus gros |
| PC:GB | 0.25*** | 3390 | Microcristaux plus gros, plus allongés et de forme plus irrégulière que pour le rapport molaire PC/GB de 1.0 |

* préparation E-39
** préparation E-35
*** préparation E-40

3. Toxicité

La préparation E-35 de microcristaux GB:PC (rapport molaire de 1,0) a été injectée en i.v. à des souris Balb/c de 22 grammes. A la dose de 118,4 mg/kg, la perte de poids maximum est de 16,2 % au jour 3 puis la souris récupère son poids initial au jour 8.

A la dose de 178,5 mg/kg, la perte de poids a été continue et la souris est morte au jour 5. Aux doses de 56,6 et 28,4 mg/kg, aucune toxicité n'est observée, on observe même un gain de poids de 6 à 8 % après 2 jours à la plus faible dose essayée.

4. Stabilité des microcristaux de GB

La préparation E-35 (rapport PC/GB de 1,0) a été conservée sous azote à 4°C pendant 100 jours et examinée en microscopie optique à contraste de phase, le jour de leur préparation, aux jours 7, 15, 20 et 100. Au jour 7, la population est relativement homogène avec une taille moyenne un peu plus élevée qu'au jour 1. Au jour 100, on observe toujours des petits microcristaux, mais également la présence de grappes de microcristaux. La mesure des tailles moyennes au moyen de nanosizer confirme les résultats. Entre le jour 1 et le jour 20, la taille moyenne a doublé et au jour 100, elle est d'environ 2 µm.

L'augmentation de la taille moyenne des microcristaux résulte donc de la formation lente d'agrégats de microcristaux dont les tailles individuelles ne semblent pas augmenter au cours du temps. Cette formation d'agrégats au cours de l'entreposage à 4°C peut être évitée par adjonction d'additifs tels que des anti-oxydants ou des saccharides.

En conclusion, on peut obtenir une population relativement homogène de microcristaux de gingkolide B, de taille comprise entre 0,1 et 1 µm par interaction spécifique et équimolaire de cet agent anti-PAF-acéther avec une molécule amphiphile comme la phosphatidylcholine. Par examen en microscopie optique, il semble que la population de microcristaux est relativement homogène au point de vue taille et forme. En microscopie électronique, ces microcristaux se présentent sous forme de "galets" aux bords arrondis.

Sans difficulté de préparation, on peut obtenir une suspension laiteuse contenant 12,8 mg en gingkolide B par ml.

La toxicité de cette préparation de microcristaux après administration i.v. chez la souris est faible. L'activité anti-PAF-acéther de cette préparation est vérifiée.

Des microcristaux sont également obtenus, si on ajoute en plus de la phosphatidylcholine,du cholestérol et de la stéarylamine (PC:Ch:SA ; 4:3:1) à condition que le rapport molaire PC/GB soit inférieur à 2.

EXEMPLE 3

AGENTS ANTI-PAF-ACETHER

On trouvera dans la littérature de nombreux autres agents antiPAF-acéther susceptibles d'être appliqués au procédé selon la présente invention, notamment dans "PAF-acéther specific binding sites : 2. Design of specific antagonists" de P. Braquet et J.J. Godfroid (TIPS - October 1986 - Elsevier Science Publishers B.V., Amsterdam).

On distingue plusieurs séries d'agents anti-PAF, entre autres :

1) les antagonistes présentant une structure moléculaire analogue à celle du PAF-acéther, telle que les CV-3988 (TAKEDA), RU-45703 (ROUSSEL-UCLAF/PHARMACOCHIMIE MOLECULAIRE), ONO-6240 (ONO), Ro 19-3704 (HOFMANN-LA ROCHE), SRI 63-119 (SANDOZ) et SRI 63-072 (SANDOZ) ou encore des antagonistes présentant une structure modifiée par rapport à celle du PAF-acéther telle que la PIPERIDINE SRI 63-073 (SANDOZ) ou le DIOXANONE (HOFFMANN-LA ROCHE).

2) des produits naturels comme les lignanes et néolignanes tels que la KADSURENONE (PIPER FUTOKADSURAE), la MAGNOSALICIDE (MAGNOLIA SALICIFOLIA), les NECTANDRINES A et B (NECTANDRA RIGIDA) et le DINORLIGNANE DE SYNTHESE L-652.731 de formule :

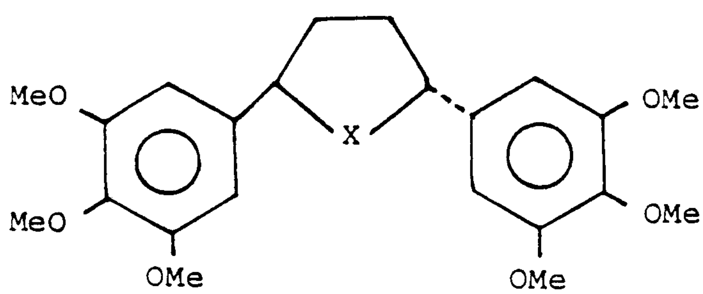

L-652.731 (X=O, MSD

Outre les terpénodes isolés du GINKGO BILOBA (GINKGOLIDES) concernés également par la présente demande de brevet.

EXEMPLE 4

PREPARATION DE MICROCRISTAUX DE KADSURENONE A PH 5,9

Des microcristaux contenant de la kadsurénone et de la phosphatidylcholine sont préparés selon le schéma suivant.

a) Préparation

Un film lipidique est formé dans un ballon en dissolvant de la phosphatidylcholine dans un solvant et la kadsurénone dans un autre solvant en quantités équimolaires, et après évaporation du solvant sous vide à l'aide d'un évaporateur rotatif à une température de 30 °C.

Ainsi, à un milligramme de kadsurénone (1 mg/ml) dans le chloroforme : méthanol (1:1 en volume), on a ajouté dans un ballon de 50 ml, 4,6 ml de phosphatidylcholine (0,5 mg/ml) dans le chloroforme : méthanol (4:1 en volume).

Les microcristaux sont formés par addition de 0,5 ml de tampon acétate 0,15 M pH 5,9 et sonication pendant 5 minutes à 100 W dans un bain à ultrasons. Les microcristaux sont conservés sous azote à 4 °C. On obtient ainsi des microcristaux ayant une taille sensiblement homogène de l'ordre de 500 nm.

b) Purification

La préparation des microcristaux est centrifugée à 2000 g pendant 15 minutes et les microcristaux sont lavés deux fois par de l'eau bidistillée. Les microcristaux dans l'eau ont le même aspect microscopique que ceux obtenus dans du tampon acétate à pH 5,9.

EXEMPLE 5

PREPARATION DE MICROCRISTAUX A PH 5,9 CONTENANT LE PRODUIT L-652.731 (MSD)

Des microcristaux contenant de la phosphatidylcholine et du produit L-652.731 (MSD) sont préparés selon le schéma décrit à l'exemple précédent. A 1mg du produit L-652.731 on ajoute 3,9 ml de phosphatidylcholine (0,5 mg/ml) dans un ballon de 50 ml et on évapore sous vide le solvant à 30 °C.

Le film est repris par 0,5 ml de tampon acétate 0,15 M, PH 5,9 et soumis aux ultrasons 5 minutes à une puissance de 100 W.

EXAMPLE 6 : Préparation de microcristaux d'amphotéricine B

Des microcristaux contenant de l'amphotéricine B et de la phosphatidylcholine dans un rapport équimolaire sort préparés selon le schéma suivant :

a) Préparation : Dans un ballon de 500 ml, on introduit 15,7 ml de phosphatidylcholine (egg lecithin 99% pure) à 10 mg/ml de chloroforme (0,2 mM). Un film lipidique est formé par évaporation du solvant sous vide à l'aide d'un évaporateur rotatif à une température de 30 °C. On ajoute ensuite 370 ml d'amphotéricine B à 0,5 mg/ml de mélange chloroforme-méthanol (1:1, en volume), soit 0,2 mM.

Après évaporation du solvant sous vide à l'aide d'un évaporateur rotatif, les microcristaux sont formés par addition de 10 ml de NaCl 9 ‰ et sonication pendant 15 min. dans un bain à ultrasons JULABO modèle USR 6.

b) Purification : La préparation de microcristaux d'amphotéricine B est centrifugée 2 minutes à 2000 g dans une centrifugeuse de table. Le surnageant est appliqué au sommet d'une colonne de Sépharose 6B éluée par du NaCl 9 ‰. La fraction du premier pic la plus enrichie en amphotéricine B contenait 8,23 mg d'amphotéricine B/ml et 7,10 mg de phosphatidylcholine/ml soit un rapport molaire ampho/PC de 0,98.

c) Toxicité aiguë chez la souris : A la dose en amphotéricine B de 82,2 mg/kg administrée en intraveineuse, la préparation de microcristaux d'amphotéricine B est toxique vis-à-vis des souris OF1 femelles, le décès de la souris intervenant dans les 3 minutes suivant l'injection. A la dose de 41,1 mg/kg, la souris injectée en intraveineuse est décédée au 2ème jour. En revanche, à la dose de 20,6 mg/kg, la souris injectée en intraveineuse perd environ 20 % de son poids durant les 7 jours suivant l'injection puis récupère son poids au jour 22.

L'amphotéricine sous forme de fungizone ® (amphotéricine B déoxycholate) administrée en intraveineuse a une $LD_{50}$ de 1,2 mg/kg selon LOPEZ-BERESTEIN et al. [2], de 2,3 mg/kg selon SZOKA et al.

[3] et de 5 mg/kg selon WRIGHT et al. [1].

La $LD_{50}$ de l'amphotéricine B-liposomes par voie intraveineuse varie de 4 à 19 mg/kg suivant la composition des liposomes (SZOKA et al., op. cit.)

La toxicité aiguë des microcristaux d'amphotéricine B-phosphatidylcholine, administrée par voie i.v. à des souris NMRI,a été comparée à celle de l'amphotéricine B complexée au déoxycholate (FUNGIZONE$^R$) et à celle de liposomes composés de phosphatidylcholine, cholestérol et de stéarylamine (rapports molaires = 4:3:1) contenant de l'amphotéricine B préparés selon la même technique qu'à l'exemple I-A)1. pour le Ginkgolide B.

La dose induisant la mort de 50 % des souris, 14 jours après l'administration unique des produits ($LD_{50}$) est de 2,5 mg/kg pour l'amphotéricine B libre ; de 18,3 mg/kg pour les microcristaux PC:AMPHO B.

Sous forme de microcristaux, l'AMPHO B est donc 7,3 fois moins toxique que sous forme de complexe au déoxycholate.

d) Cytotoxicité in vitro : La cytotoxicité in vitro est terminée par la diminution de l'activité enzymatique réductrice vis-à-vis du MTT (sel de tétrazolium) des macrophages murins J774-G8 incubés une nuit en présence de milieu RPMI enrichi de 10 % de sérum de veau foetal et contenant les produits à diverses doses.

La cytotoxicité est exprimée sous forme de la concentration micromolaire d'amphotéricine B qui réduit de 50 % l'activité enzymatique réductrice des macrophages ($TOX_{50}$).

Tableau I - Cytotoxicité des différentes formulations d'amphotéricine B

| Produit | $TOX_{50}$ (µM) |
|---|---|
| FUNGIZONE | 4,0 |
| Microcristaux PC:AMPHO | 132 |
| Microcristaux lyophylisés et reconstitués | 132 |
| Liposomes : AMPHO | 17 |

Le tableau indique que sous forme de microcristaux, l'AMPHO B est 33 fois moins toxique in vitro vis-à-vis de macrophages J774-G8 que l'AMPHO B libre et 7,7 fois moins toxique que sous forme de liposomes.

La lyophylisation des microcristaux en présence de sucrose 0,1 M et leur reconstitution n'a pas modifié la cytotoxicité de ceux-ci.

e) Activité antifungique directe (c'est-à-dire in vitro sur parasites extracellulaires) : L'activité des microcristaux d'amphotéricine B a été comparée à celle de l'amphotéricine B contrôle solubilisée dans du DMSO puis diluée dans du milieu de culture RPMI contenant 10 % de sérum déplété en lipides. Vis-à-vis de Candida Albicans et de Candida Tropicalis, la MIC (concentration minimale inhibitrice de la croissance des parasites in vitro) des 2 formes d'amphotéricine B est identique et se situe entre 0,05 et 0,1 µM (0,04 et 0,08 µg/ml).

L'activité antifungique directe de l'amphotéricine B sous forme de fungizone, de microcristaux ou de liposomes a également été mesurée sur la croissance de Candida tropicalis dans du milieu RPMI contenant 20 % de sérum de veau foetal. L'inhibition de la croissance après 16 h est observée pour des concentrations croissantes en amphotéricine B (14 concentrations allant de 0,012 à 100 µM). La concentration minimale inhibitrice (MIC) est la concentration la plus faible à laquelle on n'observe pas de croissance de Candida.

La MIC de l'amphotéricine B sous forme de microcristaux ampho B:PC ou sous forme de fungizone est identique (0,195 µM). Par contre l'amphotéricine B encapsulée dans des liposomes composés de phosphatidylcholine, cholestérol et stéarylamine dans un rapport molaire 4:3:1, est 8 fois moins active que

l'amphotéricine libre (1,56 $\mu$M).

La lyophylisation des microcristaux d'amphotéricine B:PC en présence de 0,1 M sucrose n'affecte pas l'activité antifungique de ceux-ci vis-à-vis des fungi extracellulaires.

f) Activité in vitro vis-à-vis de macrophages infectés par Candida Tropicalis (vis-à-vis de parasites intracellulaires) :

L'activité antifungique de l'amphotéricine B vis-à-vis de fungi intracellulaires est déterminée in vitro dans le modèle de la lignée J-774-G8 de macrophages infectés à raison de 1 Candida tropicalis pour 10 macrophages. Les macrophages sont cultivés dans du milieu RPMI contenant 10 mM MES pH 7,2 supplémenté par 10 % de plasma humain décomplémenté. L'action des médicaments est mesurée dans des boîtes LIMBRO à 24 puits contenant $10^5$ macrophages infectés, adhérant su des lamelles de verre, et la gamme de concentrations testées varie de 0,048 $\mu$M à 100 $\mu$M.

Le pourcentage de cellules infectées est déterminé après 16 h de coculture. Sur lames de microscope, on compte après coloration au MAY-GRUNWALT-GIEMSA, le nombre de macrophages totaux (NT) ainsi que le nombre de macrophages infectés par des Candida (Ni). Le pourcentage d'infection est le rapport Ni/NT (exprimé en %).

La concentration, exprimée en $\mu$M, qui réduit de 50 % l'infection intracellulaire ($IC_{50}$) est ensuite déterminée.

L'$IC_{50}$ de la FUNGIZONE est de 0,2 $\mu$M tandis que pour les microcristaux elle est de 0,1 $\mu$M, tandis que l'$IC_{50}$ des liposomes contenant de l'AMPHO B est de 0,8 $\mu$M.

Les microcristaux sont donc au moins aussi actifs que l'amphotéricine B, aussi bien vis-à-vis des fungi extracellulaires que vis-à-vis des fungi intracellulaires.

g) Indice thérapeutique : L'indice thérapeutique (TI), défini sur les formes intracellulaires de Candida, est le rapport entre la toxicité du produit pour les macrophages, exprimée par la $TOX_{50}$ et l'activité thérapeutique exprimée par l'$IC_{50}$, dose réduisant de 50 % l'infection intracellulaire.

Tel que l'illustre le tableau ci-dessous, l'index thérapeutique des microcristaux PC:AMPHO B est 66 fois plus élevé que celui de l'AMPHO B libre (sous forme de FUNGIZONE) et 60 fois plus élevé que celui des liposomes contenant de l'AMPHO B.

Tableau 2 : Indice thérapeutique des différentes formulations de l'amphotéricine B

| Produit | $TOX_{50}$ (µM)[a] | $IC_{50}$ (µM)[b] | TI[c] | produit / TI FUNGIZONE |
|---|---|---|---|---|
| FUNGIZONE | 4,0 | 0,2 | 20 | 1,0 |
| Microcristaux PC:AMPHO B | 132 | 0,1 | 1320 | 66,0 |
| Microcristaux lyophylisés | 132 | 0,6 | 220 | 11,0 |
| Liposomes:AMPHO | 17,0 | 0,8 | 21,3 | 1,1 |

a. $TOX_{50}$ : Concentration exprimée en µMoles, qui réduit de 50 % l'activité enzymatique réductrice des macrophages vis-à-vis des sels de tétrazolium.

b. $IC_{50}$ : Concentration, exprimée en µMoles, qui réduit de 50 % l'infection intracellulaire des macrophages J-774-G8 infectés par Candida tropicalis

c. TI : Index thérapeutique ; rapport entre la $TOX_{50}$ et l'$IC_{50}$.

EXEMPLE 7 : Préparation de microcristaux d'amphotéricine B contenant du cholestérol

L'association de l'amphotéricine B aux stérols a été tentée (WITZKE et al., [4]), en particulier au cholestérol et à l'ergostérol. L'incorporation de stérol aux liposomes contenant de l'amphotéricine B réduit leur toxicité (SZOKA et al., [3]).

L'addition de cholestérol a le même effet sur les microcristaux d'amphotéricine B tout en augmentant leur activité.

Les microcristaux d'amphotéricine B contenant de la phosphatidylcholine (PC) et du cholestérol (CH) ont été préparés selon le schéma suivant :

a) Préparation : Dans un ballon de 500 ml, on introduit 15,7 ml de phosphatidylcholine (PC) à 10 mg/ml $CHCl_3$ (0,2 mM), 370 ml d'amphotéricine B à 0,5 mg/ml de chloroforme-méthanol (1:1 en volume), soit 0,2 mM,et 58 mg de cholestérol (CH) dissous dans 10 ml de méthanol (0,15 mM).

Le solvant est évaporé sous vide à 30 °C à l'aide d'un évaporateur rotatif.

Les microcristaux sont formés par addition de 10 ml d'eau et sonication pendant 15 min. dans un bain à ultrasons JULABO modèle USR 6.

b) Purification : La préparation est purifiée comme dans l'exemple 6. La fraction du premier pic, la plus riche en amphotéricine B contient 2,55 mg ampho/ml, 9,11 mg PC/ml et 1,93 Ch/ml, soit un rapport molaire : PC/Ch/Amphotéricine B de 4/2/1.

c) Toxicité aiguë chez la souris : A la dose de 25,5 mg/kg d'amphotéricine B, administrée par voie intraveineuse à 2 souris OF1 femelles, la préparation de microcristaux d'amphotéricine B contenant du cholestérol induit une perte de poids maximale de 8 % au jour 6 chez 1 souris et un gain de poids de 9 % au jour 6 chez la 2ème souris (en moyenne : pas de perte de poids). Le poids moyen des 2 souris au jour 19 est de 100 % du poids initial.

d) Activité antifongique directe : L'activité antifongique de l'amphotéricine B contrôle (DMSO et RPMI) et la préparation de microcristaux d'amphotéricine B contenant du cholestérol sont du même ordre. La MIC se situe entre 0,05 et 0,1 µM.

e) Activité in vitro vis-à-vis de macrophages infectés : La MIC de la préparation de microcristaux d'amphotéricine B contenant du cholestérol est de 0,1 µM, tandis que pour l'amphotéricine B dissoute dans le DMSO elle est de 0,4 µM. L'amphotéricine B sous forme de microcristaux contenant du cholestérol est donc au moins 4 fois moins toxique et 4 fois plus active sur macrophages infectés par rapport à l'amphotéricine B.

REFERENCES BIBLIOGRAPHIQUES CITEES DANS LA DESCRIPTION

[1] : WRIGHT et al. The Journal of antibiotics 1982 Vol. 35 n° 7 pp 911-914.
[2] : LOPEZ-BERESTEIN et al. The Journal of infections diseases Vol. 145, n° 5, May 1983 pp 939-945.
[3] : SZOKA et al. Antimicrobial Agents and Chemotherapy, March 1987, p. 421-429 Vol. 31, n° 3.
[4] : WITZKE et al. Biochemistry 1984, 23, 1668-1674

**Revendications**

**Revendications pour les Etats contractants suivants : AT, BE, CH, DE, FR, GB, IT, LI, LU, NL, SE**

**1.** Procédé de préparation de microparticules lipidiques d'aspect cristallin d'une substance insoluble dans l'eau présentant une affinité pour les phospholipides et d'au moins un phospholipide, caractérisé en ce que :

a) on évapore le ou les solvants(s) organique(s) d'une solution de phospholipides et de ladite substance, et

b) on remet le film obtenu après évaporation du ou des solvant(s), en suspension dans une solution aqueuse par agitation vigoureuse, et le rapport molaire entre le ou les phospholipide(s) et la substance étant compris en tre 0,1 et 2.

**2.** Procédé selon la revendication 1, caractérisé en ce que le rapport molaire entre le ou les phospholipide(s) d'une part, et la substance d'autre part, est compris entre 0,8 et 1,2.

**3.** Procédé selon l'une des revendications précédentes, caractérisé en ce que le phospholipide est la phosphatidylcholine.

**4.** Procédé selon l'une des revendications précédentes, caractérisé en ce que les divers solvants des solutions utilisées sont choisis parmi les solvants organiques usuels, notamment le méthanol, le chloroforme et leurs mélanges.

**5.** Procédé selon l'une des revendications précédentes, caractérisé en ce que l'étape a) se subdivise ainsi :

$a_1$) on évapore le(s) solvant(s) de la solution de phospholipide(s),

$a_2$) on remet le film lipidique obtenu après évaporation, en solution, en ajoutant une solution de ladite substance,

$a_3$) on évapore à nouveau le(s) solvant(s) de la solution de phospholipide(s) et de ladite substance.

**6.** Procédé selon l'une des revendications précédentes, caractérisé en ce que l'agitation vigoureuse dans l'étape b) est obtenue par traitement aux ultrasons.

**7.** Procédé selon l'une des revendications précédentes, caractérisé en ce que la solution de phospholipide(s) est une solution de chloroforme ou de chloroforme/méthanol.

**8.** Procédé selon l'une des revendications précédentes, caractérisé en ce que la solution de ladite substance est une solution de chloroforme/méthanol.

**9.** Procédé selon l'une des revendications 1 à 8, caractérisé en ce que ladite substance est choisie parmi les substances antagonistes de l'acéther du "Facteur d'activation des plaquettes".

**10.** Procédé selon la revendication 9, caractérisé en ce que la substance en cause est choisie parmi les lignanes et néolignanes.

**11.** Procédé selon la revendication 9 ou 10, caractérisé en ce que la substance est choisie parmi la kadsurénone ou ses dérivés et le dinorlignane de synthèse L-652-731 et ses dérivés.

**12.** Procédé selon l'une des revendications 1 à 8, caractérisé en ce que la substance en cause est choisie parmi les ginkgolides et leurs dérivés.

**13.** Procédé selon la revendication 10, caractérisé en ce que la substance est choisie parmi les substances antagonistes du PAF-acéther présentant une structure analogue dudit PAF-acéther.

**14.** Procédé selon l'une des revendications 1 à 8, caractérisé en ce que ladite substance est choisie parmi l'amphotéricine B et ses dérivés, présentant une activité antifongique.

**15.** Procédé selon l'une des revendications précédentes, caractérisé en ce que la solution de ladite substance est additionnée de stérol(s), notamment du cholestérol et/ou de l'ergostérol.

**16.** Procédé selon la revendication 15, caractérisé en ce que le rapport molaire entre le ou les phospholipides d'une part, et le ou les stérols d'autre part, est compris entre 1 et 2.

**17.** Procédé selon l'une des revendications 9 à 13, caractérisé en ce que, dans l'étape b) du procédé, on remet le film obtenu après évaporation du solvant en suspension dans une solution tamponnée à pH compris entre 5 et 8, de préférence à pH 5,9.

**18.** Procédé selon la revendication 17, caractérisé en ce que, dans l'étape b) du procédé, on remet le film obtenu après évaporation du solvant en suspension dans une solution de tampon acétate ou phosphate.

**19.** Procédé selon l'une des revendications 14 à 16, caractérisé en ce que, dans l'étape b) du procédé, la solution aqueuse est une solution de NaCl ou de saccharide 0,1 M.

**20.** Procédé selon l'une des revendications précédentes, caractérisé en ce que, après l'étape b) du procédé, les microparticules lipidiques d'aspect cristallin sont purifiées par centrifugation et lavage à l'eau distillée ou par une solution aqueuse.

**21.** Microparticules lipidiques d'aspect cristallin obtenues notamment par la mise en oeuvre du procédé selon l'une des revendications précédentes.

**22.** Microparticules lipidiques d'aspect cristallin stables en milieu aqueux d'une substance insoluble dans l'eau et présentant une affinité pour les phospholipides et au moins un phospholipide, caractérisée en ce que le rapport molaire (phospholipide(s)/substance active) est inférieur ou égal à 2.

**23.** Microparticules lipidiques d'aspect cristallin d'une substance active choisie parmi l'amphotéricine B et ses dérivés antifongiques et au moins un phospholipide dans un rapport molaire (phospholipide(s)-/substance active) inférieur ou égal à 2.

**24.** Microparticules selon l'une des revendications 21 à 23 caractérisées en ce que le rapport molaire (phospholipide(s)/substance) est compris entre 0,8 et 1,4.

**25.** Microparticules selon l'une des revendications 21 à 24, caractérisées en ce que le phospholipide est la phosphatidylcholine ou ses dérivés.

**26.** Microparticules selon l'une des revendications 21, 22, 24 et 25, caractérisées en ce que la substance insoluble, présentant une affinité pour les phospholipides est un agent anti-PAF-acéther.

**27.** Microparticules selon la revendication 26, caractérisées en ce que la substance est choisie parmi les ginkgolides et leurs dérivés.

**28.** Microparticules selon la revendication 26, caractérisées en ce que la substance est choisie parmi les lignanes et néolignanes, telles que la kadsurénone ou ses dérivés et le dinorlignane de synthèse L-652-731 et ses dérivés.

**29.** Microparticules lipidiques d'aspect cristallin selon l'une des revendications 21 à 25, caractérisées en ce que la substance est choisie parmi l'amphotéricine B et ses dérivés antifongiques.

**30.** Microparticules lipidiques d'aspect cristallin selon l'une des revendications 21 à 29, caractérisées en ce que la substance est additionnée de stérol(s).

**31.** Microcristaux Microparticules lipidiques d'aspect cristallin selon la revendication 30, caractérisées en ce que le rapport molaire (phospholipide(s)/substance + stérol(s)) est compris entre 0,8 et 1,4.

**32.** Microcristaux Microparticules lipidiques d'aspect cristallin selon l'une des revendications 28 à 31, caractérisées en ce qu'ils comportent de l'amphotéricine B, du cholestérol et de la phosphatidylcholine dans un rapport molaire 1:2:4 respectivement.

**33.** microparticules selon l'une des revendications 21 à 32, caractérisées en ce qu'ils sont sous forme lyophylisée.

**34.** Composition pharmaceutique, caractérisée en ce qu'elle contient comme principe actif des microparticules selon l'une des revendications 21 à 33.

**35.** Composition pharmaceutique selon la revendication 34, caractérisée en ce qu'elle est conditionnée sous forme injectable ou pulvérisable.

**36.** Composition pharmaceutique selon la revendication 34, caractérisée en ce qu'elle est conditionnée sous forme lyophylisée.

**Revendications pour les Etats contractants suivants : ES, GR**

**1.** Procédé de préparation de microparticules lipidiques d'aspect cristallin d'une substance insoluble dans l'eau présentant une affinité pour les phospholipides et d'au moins un phospholipide, caractérisé en ce que :

a) on évapore le ou les solvants(s) organique(s) d'une solution de phospholipides et de ladite substance, et

b) on remet le film obtenu après évaporation du ou des solvant(s), en suspension dans une solution aqueuse par agitation vigoureuse, et le rapport molaire entre le ou les phospholipide(s) et la substance étant compris entre 0,1 et 2.

**2.** Procédé selon la revendication 1, caractérisé en ce que le rapport molaire entre le ou les phospholipide(s) d'une part, et la substance d'autre part, est compris entre 0,8 et 1,2.

**3.** Procédé selon l'une des revendications précédentes, caractérisé en ce que le phospholipide est la phosphatidylcholine ou ses dérivés.

**4.** Procédé selon l'une des revendications précédentes, caractérisé en ce que les divers solvants des solutions utilisées sont choisis parmi les solvants organiques usuels, notamment le méthanol, le chloroforme et leurs mélanges.

**5.** Procédé selon l'une des revendications précédentes, caractérisé en ce que l'étape a) se subdivise ainsi :

$a_1$) on évapore le(s) solvant(s) de la solution de phospholipide(s),

$a_2$) on remet le film lipidique obtenu après évaporation, en solution, en ajoutant une solution de ladite substance,

$a_3$) on évapore à nouveau le(s) solvant(s) de la solution de phospholipide(s) et de ladite substance.

6. Procédé selon l'une des revendications précédentes, caractérisé en ce que l'agitation vigoureuse dans l'étape b) est obtenue par traitement aux ultrasons.

7. Procédé selon l'une des revendications précédentes, caractérisé en ce que la solution de phospholipide(s) est une solution de chloroforme ou de chloroforme/méthanol.

8. Procédé selon l'une des revendications précédentes, caractérisé en ce que la solution de ladite substance est une solution de chloroforme/méthanol.

9. Procédé selon l'une des revendications 1 à 8, caractérisé en ce que ladite substance est choisie parmi les substances antagonistes de l'acéther du "Facteur d'activation des plaquettes".

10. Procédé selon la revendication 9, caractérisé en ce que la substance en cause est choisie parmi les lignanes et néolignanes.

11. Procédé selon la revendication 9 ou 10, caractérisé en ce que la substance est choisie parmi la kadsurénone ou ses dérivés et le dinorlignane de synthèse L-652-731 et ses dérivés.

12. Procédé selon l'une des revendications 1 à 8, caractérisé en ce que la substance en cause est choisie parmi les ginkgolides et leurs dérivés.

13. Procédé selon la revendication 10, caractérisé en ce que la substance est choisie parmi les substances antagonistes du PAF-acéther présentant une structure analogue dudit PAF-acéther.

14. Procédé selon l'une des revendications 1 à 8, caractérisé en ce que ladite substance est choisie parmi l'amphotéricine B et ses dérivés, présentant une activité antifongique.

15. Procédé selon l'une des revendications précédentes, caractérisé en ce que la solution de ladite substance est additionnée de stérol(s), notamment du cholestérol et/ou de l'ergostérol.

16. Procédé selon la revendication 15, caractérisé en ce que le rapport molaire entre le ou les phospholipides d'une part, et le ou les stérols d'autre part, est compris entre 1 et 2.

17. Procédé selon l'une des revendications 9 à 13, caractérisé en ce que, dans l'étape b) du procédé, on remet le film obtenu après évaporation du solvant en suspension dans une solution tamponnée à pH compris entre 5 et 8, de préférence à pH 5,9.

18. Procédé selon la revendication 17, caractérisé en ce que, dans l'étape b) du procédé, on remet le film obtenu après évaporation du solvant en suspension dans une solution de tampon acétate ou phosphate.

19. Procédé selon l'une des revendications 14 à 16, caractérisé en ce que, dans l'étape b) du procédé, la solution aqueuse est une solution de NaCl ou de saccharide 0,1 M.

20. Procédé selon l'une des revendications précédentes, caractérisé en ce que, après l'étape b) du procédé, les microparticules lipidiques d'aspect cristallin sont purifiées par centrifugation et lavage à l'eau distillée ou par une solution aqueuse.

21. Procédé de préparation de microparticules lipidiques d'aspect cristallin selon l'une des revendications précédentes, caractérisé en ce que les microparticules lipidiques d'aspect cristallin sont stables en milieu aqueux, et présentent un rapport molaire (phospholipide(s)/substance active) inférieur ou égal à 2.

22. Procédé de préparation de microparticules lipidiques d'aspect cristallin selon l'une des revendications 1 à 20, caractérisé en ce que la substance active est choisie parmi l'amphotéricine B et ses dérivés antifongiques et le rapport molaire (phospholipide(s)/substance active) est inférieur ou égal à 2.

23. Procédé selon l'une des revendications 21 à 22 caractérisé en ce que le rapport molaire (phospholipide(s)/substance) est compris entre 0,8 et 1,4.

**24.** Procédé selon l'une des revendications 21 à 23, caractérisé en ce que la substance est additionnée de stérol(s).

**25.** Procédé selon la revendication 24, caractérisé en ce que le rapport molaire (phospholipide(s)-/substance + stérol(s)) est compris entre 0,8 et 1,4.

**26.** Procédé selon l'une des revendications 24 ou 25, caractérisé en ce que l'amphotéricine B, le cholestérol et la phosphatidylcholine sont dans un rapport molaire 1:2:4 respectivement.

**27.** Procédé selon l'une des revendications 21 à 26, caractérisé en ce que les microparticules sont lyophylisées.

**28.** Procédé de préparation d'une composition pharmaceutique, caractérisé en ce que l'on mélange des microparticules obtenues par un procédé selon l'une des revendications 21 à 26 avec un véhicule pharmaceutiquement acceptable.

**29.** Procédé de préparation d'une composition pharmaceutique selon la revendication 28, caractérisé en ce qu'elle est conditionnée sous forme injectable ou pulvérisable.

**30.** Procédé de préparation d'une composition pharmaceutique selon la revendication 28, caractérisé en ce qu'elle est conditionnée sous forme lyophylisée.

**Claims**
**Claims for the following Contracting States : AT, BE, CH, DE, FR, GB, IT, LI, LU, NL, SE**

**1.** Process for the preparation of lipid microparticles of crystalline appearance comprising a substance which is insoluble in water, having an affinity for phospholipids, and at least one phospholipid, characterized in that:
a) the organic solvent(s) is(are) evaporated from a solution of phospholipids and the said substance, and
b) the film obtained after evaporation of the solvent(s) is resuspended in an aqueous solution with vigorous stirring, the molar ratio between the phospholipid(s) and the substance being between 0.1 and 2.

**2.** Process according to Claim 1, characterized in that the molar ratio between the phospholipid(s) on the one hand and the substance on the other hand is between 0.8 and 1.2.

**3.** Process according to one of the preceding claims, characterized in that the phospholipid is phosphatidylcholine.

**4.** Process according to one of the preceding claims, characterized in that the various solvents of the solutions employed are chosen from among the usual organic solvents, especially methanol, chloroform and mixtures thereof.

**5.** Process according to one of the preceding claims, characterized in that step a) is subdivided as follows:
$a_1$) the solvent(s) is(are) evaporated from the solution of phospholipid(s),
$a_2$) the lipid film obtained after evaporation is redissolved by adding a solution of the said substance,
$a_3$) the solvent(s) is(are) evaporated again from the solution of phospholipid(s) and the said substance.

**6.** Process according to one of the preceding claims, characterized in that the vigorous stirring in step b) is brought about by treatment with ultrasound.

**7.** Process according to one of the preceding claims, characterized in that the solution of phospholipid(s) is a solution in chloroform or in chloroform/methanol.

8. Process according to one of the preceding claims, characterized in that the solution of the said substance is a solution in chloroform/methanol.

9. Process according to one of Claims 1 to 8, characterized in that the said substance is chosen from substances which are antagonists of the acether of the "platelet activating factor".

10. Process according to Claim 9, characterized in that the substance in question is chosen from lignans and neolignans.

11. Process according to Claim 9 or 10, characterized in that the substance is chosen from kadsurenone or derivatives thereof and the synthetic dinorlignan L-652-731 and derivatives thereof.

12. Process according to one of Claims 1 to 8, characterized in that the substance in question is chosen from ginkgolides and derivatives thereof.

13. Process according to Claim 10, characterized in that the substance is chosen from PAF-acether antagonist substances which have a structure analogous to that of the said PAF-acether.

14. Process according to one of Claims 1 to 8, characterized in that the said substance is chosen from amphotericin B and derivatives thereof which have an antifungal activity.

15. Process according to one of the preceding claims, characterized in that sterol(s), especially cholesterol and/or ergosterol, is(are) added to the solution of the said substance.

16. Process according to Claim 15, characterized in that the molar ratio between the phospholipid(s) on the one hand and the sterol(s) on the other hand is between 1 and 2.

17. Process according to one of Claims 9 to 13 characterized in that in step b) of the process the film obtained after evaporation of the solvent is resuspended in a solution buffered at a pH of between 5 and 8, preferably at pH 5.9.

18. Process according to Claim 17, characterized in that in step b) of the process the film obtained after evaporation of the solvent is resuspended in a solution of acetate or phosphate buffer.

19. Process according to one of Claims 14 to 16 characterized in that in step b) of the process the aqueous solution is a 0.1 M saccharide or NaCl solution.

20. Process according to one of the preceding claims, characterized in that after step b) of the process the lipid microparticles of crystalline appearance are purified by centrifugation and washing with distilled water or by an aqueous solution.

21. Lipid microparticles of crystalline appearance obtained in particular by employing the process according to one of the preceding claims.

22. Lipid microparticles of crystalline appearance which are stable in aqueous medium and comprise a substance which is insoluble in water and has an affinity for phospholipids, and at least one phospholipid, characterized in that the molar ratio (phospholipid(s)/active substance) is less than or equal to 2.

23. Lipid microparticles of crystalline appearance comprising an active substance chosen from amphotericin B and antifungal derivatives thereof, and at least one phospholipid, in a molar ratio (phospholipid(s)/active substance) of less than or equal to 2.

24. Microparticles according to one of Claims 21 to 23, characterized in that the molar ratio (phospholipid(s)/substance) is between 0.8 and 1.4.

25. Microparticles according to one of Claims 21 to 24, characterized in that the phospholipid is phosphatidylcholine or derivatives thereof.

**26.** Microparticles according to one of Claims 21, 22, 24 and 25, characterized in that the insoluble substance which has an affinity for phospholipids is an anti-PAF-acether agent.

**27.** Microparticles according to Claim 26, characterized in that the substance is chosen from the ginkgolides and derivatives thereof.

**28.** Microparticles according to Claim 26, characterized in that the substance is chosen from lignans and neolignans such as kadsurenone and derivatives thereof and the synthetic dinorlignan L-652-731 and derivatives thereof.

**29.** Lipid microparticles of crystalline appearance according to one of Claims 21 to 25, characterized in that the substance is chosen from amphotericin B and antifungal derivatives thereof.

**30.** Lipid microparticles of crystalline appearance according to one of Claims 21 to 29, characterized in that sterol(s) is(are) added to the substance.

**31.** Lipid microparticles of crystalline appearance according to Claim 30, characterized in that the molar ratio (phospholipid(s)/substance + sterol(s)) is between 0.8 and 1.4.

**32.** Lipid microparticles of crystalline appearance according to one of Claims 28 to 31, characterized in that they comprise amphotericin B, cholesterol and phosphatidylcholine in a molar ratio of 1:2:4 respectively.

**33.** Microparticles according to one of Claims 21 to 32, characterized in that they are in lyophilized form.

**34.** Pharmaceutical composition, characterized in that it contains as active principle microparticles according to one of Claims 21 to 33.

**35.** Pharmaceutical composition according to Claim 34, characterized in that it is presented in injectable or pulverizable form.

**36.** Pharmaceutical composition according to Claim 34, characterized in that it is presented in lyophilized form.

**Claims for the following Contracting States : ES, GR**

**1.** Process for the preparation of lipid microparticles of crystalline appearance comprising a substance which is insoluble in water, having an affinity for phospholipids, and at least one phospholipid, characterized in that:
a) the organic solvent(s) is(are) evaporated from a solution of phospholipids and the said substance, and
b) the film obtained after evaporation of the solvent(s) is resuspended in an aqueous solution with vigorous stirring, the molar ratio between the phospholipid(s) and the substance being between 0.1 and 2.

**2.** Process according to Claim 1, characterized in that the molar ratio between the phospholipid(s) on the one hand and the substance on the other hand is between 0.8 and 1.2.

**3.** Process according to one of the preceding claims, characterized in that the phospholipid is phosphatidylcholine or derivatives thereof.

**4.** Process according to one of the preceding claims, characterized in that the various solvents of the solutions employed are chosen from among the usual organic solvents, especially methanol, chloroform and mixtures thereof.

**5.** Process according to one of the preceding claims, characterized in that step a) is subdivided as follows:
$a_1$) the solvent(s) is(are) evaporated from the solution of phospholipid(s),

$a_2$) the lipid film obtained after evaporation is redissolved by adding a solution of the said substance,
$a_3$) the solvent(s) is(are) evaporated again from the solution of phospholipid(s) and the said substance.

**6.** Process according to one of the preceding claims, characterized in that the vigorous stirring in step b) is brought about by treatment with ultrasound.

**7.** Process according to one of the preceding claims, characterized in that the solution of phospholipid(s) is a solution in chloroform or in chloroform/methanol.

**8.** Process according to one of the preceding claims, characterized in that the solution of the said substance is a solution in chloroform/methanol.

**9.** Process according to one of Claims 1 to 8, characterized in that the said substance is chosen from substances which are antagonists of the acether of the "platelet activating factor".

**10.** Process according to Claim 9, characterized in that the substance in question is chosen from lignans and neolignans.

**11.** Process according to Claim 9 or 10, characterized in that the substance is chosen from kadsurenone or derivatives thereof and the synthetic dinorlignan L-652-731 and derivatives thereof.

**12.** Process according to one of Claims 1 to 8, characterized in that the substance in question is chosen from ginkgolides and derivatives thereof.

**13.** Process according to Claim 10, characterized in that the substance is chosen from PAF-acether antagonist substances which have a structure analogous to that of the said PAF-acether.

**14.** Process according to one of Claims 1 to 8, characterized in that the said substance is chosen from amphotericin B and derivatives thereof which have an antifungal activity.

**15.** Process according to one of the preceding claims, characterized in that sterol(s), especially cholesterol and/or ergosterol, is(are) added to the solution of the said substance.

**16.** Process according to Claim 15, characterized in that the molar ratio between the phospholipid(s) on the one hand and the sterol(s) on the other hand is between 1 and 2.

**17.** Process according to one of Claims 9 to 13 characterized in that in step b) of the process the film obtained after evaporation of the solvent is resuspended in a solution buffered at a pH of between 5 and 8, preferably at pH 5.9.

**18.** Process according to Claim 17, characterized in that in step b) of the process the film obtained after evaporation of the solvent is resuspended in a solution of acetate or phosphate buffer.

**19.** Process according to one of Claims 14 to 16 characterized in that in step b) of the process the aqueous solution is a 0.1 M saccharide or NaCl solution.

**20.** Process according to one of the preceding claims, characterized in that after step b) of the process the lipid microparticles of crystalline appearance are purified by centrifugation and washing with distilled water or by an aqueous solution.

**21.** Process for the preparation of lipid microparticles of crystalline appearance according to one of the preceding claims, characterized in that the lipid microparticles of crystalline appearance are stable in aqueous medium and have a molar ratio (phospholipid(s)/active substance) of less than or equal to 2.

**22.** Process for the preparation of lipid microparticles of crystalline appearance according to one of Claims 1 to 20, characterized in that the active substance is chosen from amphotericin B and antifungal derivatives thereof and the molar ratio (phospholipid(s)/active substance) is less than or equal to 2.

**23.** Process according to one of Claims 21 and 22, characterized in that the molar ratio (phospholipid(s)-/substance) is between 0.8 and 1.4.

**24.** Process according to one of Claims 21 to 23, characterized in that sterol(s) is(are) added to the substance.

**25.** Process according to Claim 24, characterized in that the molar ratio (phospholipid(s)/substance + sterol(s)) is between 0.8 and 1.4.

**26.** Process according to one of Claims 24 and 25, characterized in that the amphotericin B, the cholesterol and the phosphatidylcholine are in a molar ratio of 1:2:4 respectively.

**27.** Process according to one of Claims 21 to 26, characterized in that the microparticles are lyophilized.

**28.** Process for the preparation of a pharmaceutical composition, characterized in that microparticles obtained by a process according to one of Claims 21 to 26 are mixed with a pharmaceutically acceptable vehicle.

**29.** Process for the preparation of a pharmaceutical composition according to Claim 28, characterized in that it is presented in injectable or pulverizable form.

**30.** Process for the preparation of a pharmaceutical composition according to Claim 28, characterized in that it is presented in lyophilized form.

**Patentansprüche**
**Patentansprüche für folgende Vertragsstaaten : AT, BE, CH, DE, FR, GB, IT, LI, LU, NL, SE**

**1.** Verfahren zur Herstellung von lipidischen Mikropartikeln mit kristallinem Aussehen einer in Wasser unlöslichen Substanz, die eine Affinität für die Phospholipide und mindestens ein Phospholipid aufweist, dadurch gekennzeichnet, daß man
a) das oder die organische(n) Lösungsmittel einer Lösung von Phospholipiden und der genannten Substanz verdampft, und
b) den nach dem Verdampfen des oder der Lösungsmittel(s) erhaltenen Film in einer wäßrigen Lösung durch kräftiges Rühren wieder in Suspension bringt, wobei das molare Verhältnis zwischen dem oder den Phospholipid(en) und der Substanz zwischen 0,1 und 2 beträgt.

**2.** Verfahren nach Anspruch 1, dadurch gekennzeichnet, daß das molare Verhältnis zwischen dem oder den Phospholipid(en) einerseits und der Substanz andererseits zwischen 0,8 und 1,2 beträgt.

**3.** Verfahren nach einem der vorstehenden Ansprüche, dadurch gekennzeichnet, daß das Phospholipid Phosphatidylcholin ist.

**4.** Verfahren nach einem der vorstehenden Ansprüche, dadurch gekennzeichnet, daß die verschiedenen Lösungsmittel der verwendeten Lösungen unter den gebräuchlichen organischen Lösungsmitteln ausgewählt werden, insbesondere Methanol, Chloroform und ihren Mischungen.

**5.** Verfahren nach einem der vorstehenden Ansprüche, dadurch gekennzeichnet, daß sich die Stufe a) wie folgt unterteilt:
$a_1$) man verdampft das (die) Lösungsmittel der Lösung des (der) Phospholipide(s),
$a_2$) man bringt den nach dem Verdampfen erhaltenen lipidischen Film wieder in Lösung, indem man eine Lösung der genannten Substanz hinzugibt,
$a_3$) man verdampft von neuem das (die) Lösungsmittel der Lösung von Phospholipid(en) und der genannten Substanz.

**6.** Verfahren nach einem der vorstehenden Ansprüche, dadurch gekennzeichnet, daß das kräftige Rühren in der Stufe b) durch Ultraschall-Behandlung erhalten wird.

7. Verfahren nach einem der vorstehenden Ansprüche, dadurch gekennzeichnet, daß die Lösung des(r) Phospholipide(s) eine Lösung von Chloroform oder Chloroform/Methanol ist.

8. Verfahren nach einem der vorstehenden Ansprüche, dadurch gekennzeichnet, daß die Lösung der genannten Substanz eine Lösung von Chloroform/Methanol ist.

9. Verfahren nach einem der Ansprüche 1 bis 8, dadurch gekennzeichnet, daß die genannte Substanz unter den antagonistischen Substanzen des Acethers des "Aktivierungsfaktors der Plättchen" ausgewählt wird.

10. Verfahren nach Anspruch 9, dadurch gekennzeichnet, daß die in Frage kommende Substanz unter den Lignanen und Neolignanen ausgewählt wird.

11. Verfahren nach Anspruch 9 oder 10, dadurch gekennzeichnet, daß die Substanz unter Kadsurenon oder seinen Derivaten und Synthese-Dinorlignan L-652-731 und seinen Derivaten ausgewählt wird.

12. Verfahren nach einem der Ansprüche 1 bis 8, dadurch gekennzeichnet, daß die in Frage kommende Substanz unter den Ginkgoliden und ihren Derivaten ausgewählt wird.

13. Verfahren nach Anspruch 10, dadurch gekennzeichnet, daß die Substanz unter den antagonistischen Substanzen des PAF-Acethers ausgewählt wird, die eine analoge Struktur des genannten PAF-Acethers aufweisen.

14. Verfahren nach einem der Ansprüche 1 bis 8, dadurch gekennzeichnet, daß die genannte Substanz unter Amphotericin B und seinen Derivaten ausgewählt wird, die eine antifungische Aktivität aufweisen.

15. Verfahren nach einem der vorstehenden Ansprüche, dadurch gekennzeichnet, daß der Lösung der genannten Substanz Sterol(e), insbesondere Cholesterol und/oder Ergosterol, zugesetzt werden.

16. Verfahren nach Anspruch 15, dadurch gekennzeichnet, daß das molare Verhältnis zwischen dem oder den Phospholipid(en) einerseits und dem oder den Sterol(en) andererseits zwischen 1 und 2 beträgt.

17. Verfahren nach einem der Ansprüche 9 bis 13, dadurch gekennzeichnet, daß man in Stufe b) des Verfahrens den nach dem Verdampfen des Lösungsmittels erhaltenen Film in einer Lösung in Suspension bringt, die auf einen pH-Wert zwischen 5 und 8, vorzugsweise auf pH 5,9, gepuffert ist.

18. Verfahren nach Anspruch 17, dadurch gekennzeichnet, daß man in Stufe b) des Verfahrens den nach dem Verdampfen des Lösungsmittels erhaltenen Film in einer Lösung von Acetat- oder Phosphat-Puffer in Suspension bringt.

19. Verfahren nach einem der Ansprüche 14 bis 16, dadurch gekennzeichnet, daß in Stufe b) des Verfahrens die wäßrige Lösung eine 0,1 M Lösung von NaCl oder Saccharid ist.

20. Verfahren nach einem der vorstehenden Ansprüche, dadurch gekennzeichnet, daß nach Stufe b) des Verfahrens die lipidischen Mikropartikeln mit kristallinem Aussehen durch Zentrifugieren und Waschen mit destilliertem Wasser oder durch eine wäßrige Lösung gereinigt werden.

21. Lipidische Mikropartikel mit kristallinem Aussehen, insbesondere erhalten durch die Anwendung des Verfahrens nach einem der vorstehenden Ansprüche.

22. Lipidische Mikropartikel mit kristallinem Aussehen, die im wäßrigen Medium einer in Wasser unlöslichen Substanz stabil sind und eine Affinität für die Phospholipide und mindestens ein Phospholipid aufweisen, dadurch gekennzeichnet, daß das molare Verhältnis (Phospholipid(e)/aktive Substanz) unterhalb von oder gleich 2 beträgt.

23. Lipidische Mikropartikel mit kristallinem Aussehen einer aktiven Substanz, ausgewählt unter Amphotericin B und seinen antifungischen Derivaten, und mindestens einem Phospholipid in einem molaren Verhältnis (Phospholipid(e)/aktive Substanz) von unterhalb oder gleich 2.

**24.** Mikropartikel nach einem der Ansprüche 21 bis 23, dadurch gekennzeichnet, daß das molare Verhältnis (Phospholipid(e)/Substanz) zwischen 0,8 und 1,4 liegt.

**25.** Mikropartikel nach einem der Ansprüche 21 bis 24, dadurch gekennzeichnet, daß das Phospholipid Phosphatidylcholin oder eines seiner Derivate ist.

**26.** Mikropartikel nach einem der Ansprüche 21, 22, 24 und 25, dadurch gekennzeichnet, daß die unlösliche Substanz, die eine Affinität für die Phospholipide aufweist, ein Anti-PAF-Acether-Mittel ist.

**27.** Mikropartikel nach Anspruch 26, dadurch gekennzeichnet, daß die Substanz unter den Ginkgoliden und ihren Derivaten ausgewählt wird.

**28.** Mikropartikel nach Anspruch 26, dadurch gekennzeichnet, daß die Substanz unter den Lignanen und Neolignanen, wie Kadsurenon oder seinen Derivaten und Synthese-Dinorlignan L-652-731 und seinen Derivaten ausgewählt wird.

**29.** Lipidische Mikropartikel mit kristallinem Aussehen nach einem der Ansprüche 21 bis 25, dadurch gekennzeichnet, daß die Substanz unter Amphotericin B und seinen antifungischen Derivaten ausgewählt wird.

**30.** Lipidische Mikropartikel mit kristallinem Aussehen nach einem der Ansprüche 21 bis 29, dadurch gekennzeichnet, daß der Substanz Sterol(e) zugesetzt wird (werden).

**31.** Lipidische Mikropartikel mit kristallinem Aussehen nach Anspruch 30, dadurch gekennzeichnet, daß das molare Verhältnis (Phospholipid(e)/Substanz + Sterol(e)) zwischen 0,8 und 1,4 liegt.

**32.** Lipidische Mikropartikel mit kristallinem Aussehen nach einem der Ansprüche 28 bis 31, dadurch gekennzeichnet, daß sie Amphotericin B, Cholesterol und Phosphatidylcholin in einem molaren Verhältnis von 1:2:4 umfassen.

**33.** Mikropartikel nach einem der Ansprüche 21 bis 32, dadurch gekennzeichnet, daß sie in lyophilisierter Form vorliegen.

**34.** Pharmazeutische Zusammensetzung, dadurch gekennzeichnet, daß sie als Wirkstoff Mikropartikel nach einem der Ansprüche 21 bis 33 enthält.

**35.** Pharmazeutische Zusammensetzung nach Anspruch 34, dadurch gekennzeichnet, daß sie in injizierbarer oder versprühbarer Form konditioniert wird.

**36.** Pharmazeutische Zusammensetzung nach Anspruch 34, dadurch gekennzeichnet, daß sie in lyophilisierter Form konditioniert wird.

**Patentansprüche für folgende Vertragsstaaten : ES, GR**

**1.** Verfahren zur Herstellung von lipidischen Mikropartikeln mit kristallinem Aussehen einer in Wasser unlöslichen Substanz, die eine Affinität für die Phospholipide und mindestens ein Phospholipid aufweist, dadurch gekennzeichnet, daß man

a) das oder die organische(n) Lösungsmittel einer Lösung von Phospholipiden und der genannten Substanz verdampft, und

b) den nach dem Verdampfen des oder der Lösungsmittel(s) erhaltenen Film in einer wäßrigen Lösung durch kräftiges Rühren wieder in Suspension bringt, wobei das molare Verhältnis zwischen dem oder den Phospholipid(en) und der Substanz zwischen 0,1 und 2 beträgt.

**2.** Verfahren nach Anspruch 1, dadurch gekennzeichnet, daß das molare Verhältnis zwischen dem oder den Phospholipid(en) einerseits und der Substanz andererseits zwischen 0,8 und 1,2 beträgt.

**3.** Verfahren nach einem der vorstehenden Ansprüche, dadurch gekennzeichnet, daß das Phospholipid Phosphatidylcholin oder seine Derivate ist.

4. Verfahren nach einem der vorstehenden Ansprüche, dadurch gekennzeichnet, daß die verschiedenen Lösungsmittel der verwendeten Lösungen unter den gebräuchlichen organischen Lösungsmitteln ausgewählt werden, insbesondere Methanol, Chloroform und ihren Mischungen.

5. Verfahren nach einem der vorstehenden Ansprüche, dadurch gekennzeichnet, daß sich die Stufe a) wie folgt unterteilt:
   $a_1$) man verdampft das (die) Lösungsmittel der Lösung des (der) Phospholipide(s),
   $a_2$) man bringt den nach dem Verdampfen erhaltenen lipidischen Film wieder in Lösung, indem man eine Lösung der genannten Substanz hinzugibt,
   $a_3$) man verdampft von neuem das (die) Lösungsmittel der Lösung von Phospholipid(en) und der genannten Substanz.

6. Verfahren nach einem der vorstehenden Ansprüche, dadurch gekennzeichnet, daß das kräftige Rühren in der Stufe b) durch Ultraschall-Behandlung erhalten wird.

7. Verfahren nach einem der vorstehenden Ansprüche, dadurch gekennzeichnet, daß die Lösung des(r) Phospholipide(s) eine Lösung von Chloroform oder Chloroform/Methanol ist.

8. Verfahren nach einem der vorstehenden Ansprüche, dadurch gekennzeichnet, daß die Lösung der genannten Substanz eine Lösung von Chloroform/Methanol ist.

9. Verfahren nach einem der Ansprüche 1 bis 8, dadurch gekennzeichnet, daß die genannte Substanz unter den antagonistischen Substanzen des Acethers des "Aktivierungsfaktors der Plättchen" ausgewählt wird.

10. Verfahren nach Anspruch 9, dadurch gekennzeichnet, daß die in Frage kommende Substanz unter den Lignanen und Neolignanen ausgewählt wird.

11. Verfahren nach Anspruch 9 oder 10, dadurch gekennzeichnet, daß die Substanz unter Kadsurenon oder seinen Derivaten und Synthese-Dinorlignan L-652-731 und seinen Derivaten ausgewählt wird.

12. Verfahren nach einem der Ansprüche 1 bis 8, dadurch gekennzeichnet, daß die in Frage kommende Substanz unter den Ginkgoliden und ihren Derivaten ausgewählt wird.

13. Verfahren nach Anspruch 10, dadurch gekennzeichnet, daß die Substanz unter den antagonistischen Substanzen des PAF-Acethers ausgewählt wird, die eine analoge Struktur des genannten PAF-Acethers aufweisen.

14. Verfahren nach einem der Ansprüche 1 bis 8, dadurch gekennzeichnet, daß die genannte Substanz unter Amphotericin B und seinen Derivaten ausgewählt wird, die eine antifungische Aktivität aufweisen.

15. Verfahren nach einem der vorstehenden Ansprüche, dadurch gekennzeichnet, daß der Lösung der genannten Substanz Sterol(e), insbesondere Cholesterol und/oder Ergosterol, zugesetzt werden.

16. Verfahren nach Anspruch 15, dadurch gekennzeichnet, daß das molare Verhältnis zwischen dem oder den Phospholipid(en) einerseits und dem oder den Sterol(en) andererseits zwischen 1 und 2 beträgt.

17. Verfahren nach einem der Ansprüche 9 bis 13, dadurch gekennzeichnet, daß man in Stufe b) des Verfahrens den nach dem Verdampfen des Lösungsmittels erhaltenen Film in einer Lösung in Suspension bringt, die auf einen pH-Wert zwischen 5 und 8, vorzugsweise auf pH 5,9, gepuffert ist.

18. Verfahren nach Anspruch 17, dadurch gekennzeichnet, daß man in Stufe b) des Verfahrens den nach dem Verdampfen des Lösungsmittels erhaltenen Film in einer Lösung von Acetat- oder Phosphat-Puffer in Suspension bringt.

19. Verfahren nach einem der Ansprüche 14 bis 16, dadurch gekennzeichnet, daß in Stufe b) des Verfahrens die wäßrige Lösung eine 0,1 M Lösung von NaCl oder Saccharid ist.

20. Verfahren nach einem der vorstehenden Ansprüche, dadurch gekennzeichnet, daß nach Stufe b) des Verfahrens die lipidischen Mikropartikeln mit kristallinem Aussehen durch Zentrifugieren und Waschen mit destilliertem Wasser oder durch eine wäßrige Lösung gereinigt werden.

21. Verfahren zur Herstellung von lipidischen Mikropartikeln mit kristallinem Aussehen nach einem der vorstehenden Ansprüche, dadurch gekennzeichnet, daß die lipidischen Mikropartikeln mit kristallinem Aussehen im wäßrigen Medium stabil sind und ein molares Verhältnis (Phospholipid(e)/aktive Substanz) von unterhalb oder gleich 2 aufweisen.

22. Verfahren zur Herstellung von lipidischen Mikropartikeln mit kristallinem Aussehen nach einem der Ansprüche 1 bis 20, dadurch gekennzeichnet, daß die aktive Substanz unter Amphotericin B und seinen antifungischen Derivaten ausgewählt wird und das molare Verhältnis (Phospholipid(e)/aktive Substanz) unterhalb von oder gleich 2 beträgt.

23. Verfahren nach einem der Ansprüche 21 bis 22, dadurch gekennzeichnet, daß das molare Verhältnis (Phospholipid(e)/Substanz) zwischen 0,8 und 1,4 liegt.

24. Verfahren nach einem der Ansprüche 21 bis 23, dadurch gekennzeichnet, daß der Substanz Sterol(e) zugesetzt wird (werden).

25. Verfahren nach Anspruch 24, dadurch gekennzeichnet, daß das molare Verhältnis (Phospholipid(e)-/Substanz + Sterol(e)) zwischen 0,8 und 1,4 liegt.

26. Verfahren nach einem der Ansprüche 24 oder 25, dadurch gekennzeichnet, daß Amphotericin B, Cholesterol und Phosphatidylcholin in einem molaren Verhältnis von 1:2:4 vorliegen.

27. Verfahren nach einem der Ansprüche 21 bis 26, dadurch gekennzeichnet, daß die Mikropartikel lyophilisiert werden.

28. Verfahren zur Herstellung einer pharmazeutischen Zusammensetzung, dadurch gekennzeichnet, daß man die nach einem Verfahren gemäß einem der Ansprüche 21 bis 26 erhaltenen Mikropartikel mit einem pharmazeutisch akzeptablen Träger vermischt.

29. Verfahren zur Herstellung einer pharmazeutischen Zusammensetzung nach Anspruch 28, dadurch gekennzeichnet, daß sie in injizierbarer oder versprühbarer Form konditioniert wird.

30. Verfahren zur Herstellung einer pharmazeutischen Zusammensetzung nach Anspruch 28, dadurch gekennzeichnet, daß sie in lyophilisierter Form konditioniert wird.